# EUROPEAN PATENT APPLICATION

(11) **EP 4 353 270 A1**
(43) Date of publication of application: **17.04.2024**
(21) Application number: 23184268.3
(22) Date of filing: 07.07.2023
(51) Int. Cl.: A61L 9/03, A61L 9/12, B60H 3/00

(54) **FRAGRANCE DISPENSER SYSTEMS FOR USE WITH FLAMELESS CANDLES**

(30) Priority: 08.07.2022 US 202263367964 P
(71) Applicant: Young March Co., Ltd, Irvine, California 92603 (US)
(72) Inventor: CHIANG, Hsiu Ching, Irvine, 92603 (US); RUSHING, Alan Douglas, Irvine, 92603 (US); CHANG, Hui Shu, Irvine, 92603 (US)
(74) Representative: Murgitroyd & Company

(57) **Abstract**

Fragrance dispenser systems are disclosed. The system can include a flameless candle, such as an LED candle, and a base. The base can be configured to receive and support the flameless candle. The base can include a fan unit and a fragrance cartridge. The fan unit can blow air over the fragrance cartridge. The fragranced air can be discharged from the base into the ambient environment.

## Description

### CROSS REFERENCE

This application claims the priority benefit of U.S. Patent Application No. 63/367,964, filed July 8, 2022, which is hereby incorporated by reference herein in its entirety.

### BACKGROUND

### Field

The present disclosure relates generally to fragrance dispenser systems for use with flameless candles or items of décor such as vases, figurines, etc., or as a stand-alone unit.

### Description of Certain Related Art

Fragrance is an important part of an environment. Traditionally, candles have been a primary way of providing fragrance to an environment, such as room in a home, office, or business. When the candle is lit, the flame melts some of the candle wax and fragrance embedded in the wax is released and lifted into the environment, due to the airflow in the vicinity of the flame. However, candles can be dangerous and messy.

Some people opt to use flameless candles (also called electronic candles), which are devices that look like a traditional candle but use an electric light, such as an LED, to provide illumination. Some flameless candles may include a cylindrical body and an LED concealed within a flame shaped cap, known as a "flamecap." Some flameless candles have a "moving flame," which is a flame-shaped piece of plastic that is illuminated from a concealed LED. The relevant components (such as battery, printed circuit board, wiring, etc.) all need to be contained within a cavity of the flameless candle's outer shell.

### SUMMARY OF CERTAIN ASPECTS

Flameless candles have achieved many visual attributes of traditional candles, but have not been able to adequately address the issue of delivering fragrance.

To dispense fragrance, valuable space must be allocated inside the flameless candle for the fragrance dispensing elements. For example, space may be required to hold some form of fragrance cartridge, including an access panel to insert and remove the cartridge. Additionally, a mechanism to accelerate the fragrance out of the flameless candle may be required, such as a fan or heating element. Further, there may be a need for some form of pass-through ventilation, such as elevating the flameless candle via stand-offs or legs on the base and vents in the upper section of the flameless candle, to allow the fragrance to exit. Further, when fragrance components are located in the flameless candle, if the flameless candle's outer shell is translucent such components may be visible during illumination of the candle, or if the outer shell is relatively opaque in the vicinity of such components (e.g., to hide such components from view) a darkened region may be visible during illumination of the candle.

Flameless candles have significant drawbacks that limit their ability to effectively dispense fragrance. One drawback is the insufficient airflow needed to achieve effective dispensing of the fragrance. This is due to the congested layout in the interior of the flameless candle to house the batteries, printed circuit board (PCB), wiring, lighting elements, fragrance cartridge, fan, heater, and other components. Another drawback is the extra power needed to operate the fan or heater causes a loss of battery run time for the flameless candle. A further drawback is the presence of ventilation holes on the top surface of the candle, which are needed to allow the fragrance to leave the flameless candle but are not aesthetically pleasing and/or visibly reveal that the flameless candle is not a "real" (wax) candle. Aspects of the present disclosure seek to address one or more of the concerns discussed above, or other concerns.

In various embodiments, a fragrance dispenser includes a base (e.g., a plate or holder) for use with a flameless candle. The base can be positioned below the flameless candle and can be a separate unit from the flameless candle. For example, the flameless candle can sit on the base and is able to be readily lifted off the base by a user (e.g., vertically). The base can be unattached to the flameless candle, such as there being no latch or twist and lock feature for securing the base and flameless candle. The flameless candle can be separable from the base. As discussed in more detail below, the base can be configured to support the flameless candle and to dispense fragrance into the ambient environment. In various embodiments, the flameless candle does not dispense fragrance and/or does not contain fragrance dispensing elements. In several embodiments, the base operates separate from the flameless candle. For example, the base can dispense fragrance without the flameless candle and/or no components of the base are present in the flameless candle and/or the flameless candle does not include any fragrance dispensing mechanisms or components. In several implementations, fragranced air is not passed through the flameless candle. In various embodiments, the components that provide fragrance and/or that draw-in air to be fragranced and/or that exhaust fragranced air (e.g., a fan and motor) are not contained within a cavity of the flameless candle's outer shell. In several implementations, the base can operate (e.g., drive a fan, dispense fragrance, etc.) without the flameless candle.

The base can include an outer cover. The outer cover can have various shapes, such as circular or generally square in shape in a horizontal orientation. The outer cover can include a top panel and at least one side panel. Some implementations include a plurality (e.g., two three, four, five, or more) of side panels. The side panels can include corners that extend downward, for example, the corners can make, or nearly make, contact with a surface on which the base is placed. The top panel can include an indentation, which is configured to receive a bottom of the flameless candle. The indentation can be, for example, circular, square, rectangular, hexagonal, octagonal or other shapes. The indentation can be shaped to generally correspond to the shape of an outer periphery of the flameless candle. For example, in some embodiments, the indentation is circular with approximately a 3 inch diameter and is configured to receive a 3 inch diameter flameless candle. In some embodiments, the base does not include a feature that is received in and/or protrudes up into the flameless candle. For example, in certain embodiments, the base does not include a raised wall that is inserted into the cavity of flameless candle. Various embodiments of the base do not impede or alter the illumination provided by the flameless candle. In some embodiments, the center of the top panel can have a recessed circular impression sized to allow placement of a flameless candle, such as a 3 inch or 4 inch diameter pillar flameless candle.

The base can house various components for controlling and/or dispensing fragrance. Moving components out of the flameless candle and into the base can address, for example, problems associated with space constraints in the flameless candle. The base can include a PCB configured to control off/on, various timing features, or other aspects. The base can include a fan to force air and/or fragrance out of the base. The base can include a power source. The power source can include, for example, one or more batteries (e.g., rechargeable batteries), a removable A/C power source, a hard-wired A/C power source, inductive power, wireless rechargeable batteries, solar cells, and/or otherwise. Some implementations have a power port (micro USB, A/C, etc.) to supply power to device and/or to the rechargeable batteries. The port can be on a side panel opposite of the tray. Wiring can connect the fan, power port, power switch, and/or power source.

The base can be configured to intake ambient air. For example, the base can be elevated, such as with standoffs or legs, to facilitate airflow under the device and into the base. Air intake vents can be positioned near or immediately adjacent to the fan. In some embodiments, a horizontal edge of each side panel between each corner is scalloped to aid in airflow to the intake vent located on the bottom of the fragrance dispenser.

The base can include a fragrance-dispensing element. For example, a fragrance cartridge can be inserted into a sliding tray on one side of the fragrance dispensing unit. The fragrance cartridge can have a unique shape that ensures the cartridge is installed in the correct orientation in the tray. The fragrance cartridge can be an independent unit from and/or separable from the fragrance dispensing device. The tray can be located on one of the side panels. The tray can be configured to enable a user to insert and remove the fragrance cartridge. In some embodiments, below the tray is a finger hole to allow access to the tray door to pull the tray out. The tray can have detents, latches, or other securing elements to help secure the tray in position when fully inserted into the fragrance dispenser.

The fan can force air over, around, and/or through the tray and/or the fragrance cartridge. The fragranced air can be directed via ducts to exhaust vents in the outer cover that dispense the fragranced air into the ambient environment. In some embodiments, the vents are located in corners of the top panel. The vents can be slotted, grated, or otherwise. An interior side of the outer cover can have vanes that direct the air flow from the fan towards each corner where the air can exhaust through the vents.

The base can include a first support element, such as a base plate. The base plate can fit inside the outer cover. The base plate does not need to be the bottommost part of the base. In some embodiments, in a center of the base plate is the intake vent that the allows air to be drawn into the base by the fan. The base plate can have an opening for a power switch. The base plate and outer cover can be coupled, such as with fasteners (e.g., screws). In some embodiments, each corner of the based includes a positioning tube to locate where the fasteners go to attach the outer cover. The positioning tubes can act as stand-offs as is or may use small rubber inserts, which can cover the screw and act as a non-skid stand-off.

The base can include a second support element, such as a middle plate. The middle plate can fit inside the outer cover. The middle plate can be positioned between the outer cover and the base plate. The middle plate does not need to be in the exact middle of the base. The fan can be positioned, for example, in the center of the bottom side of the middle plate. The rechargeable batteries and/or the PCB can be located along a perimeter of the bottom side of the middle plate. A power switch can be attached to the PCB and positioned so that it aligns with a slot in the base plate. A power port on the PCB can be positioned so that it aligns with the slot in the side panel of the outer cover for the power port. A top side of the middle plate can include guides to contain the fragrance cartridge tray. The guides can position the fragrance cartridge directly over the fan. In certain implementations, the rechargeable batteries are located between the base plate and the middle plate. The middle plate can be secured (e.g., directly) to the base plate, such as by fasteners (e.g., screws). The middle plate can have holes, such as at corners of the middle plate. In some embodiments, the fasteners that secure the base plate to the outer cover can pass through the holes in the middle plate.

The fragrance dispenser may have a range of operating modes. For example, the fragrance dispenser can have an off/on mode operated via the power switch. Another mode may include a timer mode, which allows the unit to turn on at a specified time, run for a designated period (such as several hours), then turn off. The cycle may repeat, such as every 24 hours. Another mode is a "pulse" mode. In this mode, the fan will turn on at more frequent intervals (such as on for 1 minute then off for 14 minutes). The pulse mode allows a burst of fragrance to be dispensed at short intervals, which allows a consumer to better sense the fragrance rather than become acclimated to it to the point they no longer sense it and/or provides a positive benefit to overall battery run time as the fan is only operating a few minutes per hour.

Certain implementations incorporate remote control technology to allow the fragrance dispensing device to be operated remotely. For example, some implementations include a remote control that is configured to remotely communicate with and control the fragrance dispenser. The remote control can be, for example, an infrared remote, a radiofrequency remote, or otherwise. In some variants, the remote control can be programmed to control a remotely-controllable flameless candle and/or the fragrance dispenser can be configured to be controlled by a flameless candle's remote control.

The outer cover, base plate, middle plate and fragrance tray can be molded in a variety of plastics, such as ABS, acrylic, polypropylene, etc. The outer cover can be molded in an appealing color, such that the fragrance dispensing device will have neutral aesthetics.

Some implementations include a decorative cover. The cover can be configured to fit snuggly over the top of the base. This can enable a user to easily vary the visual appearance of the fragrance dispenser. For example, the fragrance dispenser can be adapted to take on visual aesthetics to match a range of décor. The cover can be large enough accommodate a 4" candle. The cover can include openings in alignment with the exhaust vents, so as not to impede the flow of fragranced air as well as preserving the orientation position for placing a flameless candle in the recessed circular impression on the outer cover.

In some implementations, a fragrance dispenser system comprises a flameless candle and a base. The flameless candle can have a longitudinal axis and a bottom. The flameless candle can include an LED candle. The flameless candle can be removably rested on the base. The base can be configured to receive and support the flameless candle.

The base can include an upper surface comprising a recess and one or a plurality of outlet vents. The outlet vents can exhaust air into the ambient environment. The recess can be shaped to correspond to the shape of the bottom of the flameless candle. The base can include a lower surface comprising one or a plurality of inlet vents. The base can include a sidewall connecting the upper surface and the lower surface. The sidewall can include a tray. The base can include a fragrance cartridge configured to be removably received in the tray. The base can include a fan. The fan can be positioned beneath the tray. The base can include an electric motor operably connected with the fan.

The base can be configured to draw air into the base through the inlet vent. The base can be configured to pass the air through the tray. The base can be configured to dispense the air out of the base through the plurality of outlet vents in a direction generally parallel to the longitudinal axis of the flameless candle and/or away from a surface that the base is resting on and/or toward a room ceiling. Some implementations do not dispense air laterally (e.g., sideways) or from a sidewall of the base.

The fragrance dispenser system can include a power source. The power source can include a battery. The battery can be rechargeable. The base can include a power port to receive a power plug to facilitate charging of the batteries.

The sidewall can include an opening. The tray can be configured to move through the opening between opened and closed positions. The tray can move by sliding, rotating, or otherwise. The fragrance cartridge can be specially configured to fit in the tray. The fragrance cartridge can include a keyed opening. The fragrance cartridge can be a solid. In some variants, the fragrance cartridge comprises a liquid.

The fragrance dispenser system can include a cover. The cover can be configured to removably engage the housing of the base. The cover can enable a user to alter the external visual appearance of the base. The cover can include one or more clips to secure the housing of the base.

The base can include a controller, such as a printed circuit board, configured to control the electric motor. The controller can be programmed to turn on and off the fan. The controller can be programmed to control a period of operation of the fragrance dispenser system.

The base can include a heater, such as an electric resistance heater. The heater can be configured to heat the fragrance cartridge. The controller can be programmed to control the heater.

In some implementations, a fragrance dispenser system includes a base and a solid fragrance cartridge. The base can include an upper surface comprising a planar region. The planar region can be configured to receive a flameless candle. For example, the planar region can be configured to support a 3 inch or greater diameter of the flameless candle.

The upper surface can include one or a plurality of outlet vents. The one or more outlet vents can be configured to blow air in a direction generally perpendicular to the planar region. The outlet vents can be distributed (e.g., approximately equally spaced) around the base and/or the periphery of the flameless candle, which can facilitate generally even fragrance distribution in the vicinity of the fragrance dispenser system.

The base can include a lower surface comprising one or a plurality of inlet vents. The base can include a sidewall connecting the upper surface and the lower surface. The sidewall can include an opening. The base can include a tray configured to move (e.g., slide) in and out of the opening, such as between a first position and a second position. The tray can be configured to removably receive the cartridge.

The base can include a fan. The base can include an electric motor operably connected with the fan. The fan can be positioned beneath the tray. The base can include a printed circuit board configured to control the electric motor. The inlet vent can be configured to receive air from below the fragrance cartridge. The fan can be configured to blow air upward onto the solid fragrance cartridge positioned in the tray.

A fragrance dispenser system can include a base, and a cover configured to be removably installed over the base.

The base can include an upper surface comprising a receiving region and an outlet vent. The receiving region can be configured to receive the bottom of a flameless candle. The base can include a lower surface comprising an inlet vent. The base can include a sidewall connecting the upper surface and the lower surface, the sidewall comprising an opening. The base can include a tray configured to be received in the opening. The base can include a fan configured to blow air out of the outlet vent. The base can include an electric motor operably connected with the fan.

The cover can include an upper surface comprising a first aperture and a second aperture. The cover can include a cover wall that extends downward from the upper surface. The cover can be configured such that, when the cover is installed over the base, the cover wall of the cover overlaps the sidewall of base, such that the cover wall blocks view of the sidewall.

The cover can be configured such that, when the cover is installed over the base, the first aperture of the cover corresponds to the receiving region of the base, such that the bottom of the flameless candle can be passed through the first aperture and received on the receiving region. The cover can be configured such that, when the cover is installed over the base, the second aperture of the cover corresponds to the outlet vent of the base, such that the air blown out of the outlet vent passes through the second aperture.

The fragrance dispenser system can include a fragrance cartridge configured to be received in the base, such as in the tray. The fragrance cartridge can be a solid fragrance cartridge. The fragrance cartridge can be made of diatomaceous earth, scented polymer, liquid and/or porous membrane, wicking fiber, or otherwise. The fragrance cartridge can include a circular hole with a notch to one side.

The discussion above is provided to summarize certain non-limiting aspects of the present technology and does not limit or define the scope of protection.

### BRIEF DESCRIPTION OF THE DRAWINGS

Certain features, aspects, and advantages of the subject matter disclosed herein are described below with reference to the drawings, which are intended to illustrate and not to limit the scope of the disclosure. Various features of different disclosed embodiments can be combined to form additional embodiments, which are part of this disclosure. No structures, features, steps, or processes are essential or critical; any can be omitted in certain embodiments. The figures are drawn to scale, but such scale should not be limiting, since dimensions and proportions other than what are shown are contemplated and are within the scope of this disclosure. Distances, angles, etc. are merely illustrative and do not necessarily bear an exact relationship to actual dimensions and layout of the devices illustrated.
Figure 1 is a perspective view of a first embodiment of a fragrance dispenser system comprising a flameless (e.g., LED) candle and a base.
Figure 2 is a perspective view of the base of Figure 1.
Figure 3 is an exploded view of certain components of the base of Figure 1, including an outer cover, tray, fan unit, and fragrance cartridge.
Figure 4 is a perspective view of the base of Figure 1, showing the tray, fan unit, and fragrance cartridge within the base in dotted lines.
Figure 5 is a top perspective view of a base plate of the base of Figure 1.
Figure 6 is a bottom perspective view of a middle plate of the base of Figure 1.
Figure 7 is a partially exploded view of the middle plate.
Figure 8 is an exploded view of a support assembly, including the base plate of Figure 4 and the middle plate of Figure 5, and the tray.
Figure 9 is a perspective view of the support assembly and the tray.
Figure 10 is a perspective view of the support assembly and the tray, showing an outlet air flow.
Figure 11 is a perspective view of the fragrance cartridge.
Figure 12 is a perspective view the support assembly and tray with the fragrance cartridge.
Figure 13 is a perspective view of the system of Figure 1, with an outlet air flow from the base in a direction generally parallel to a longitudinal axis of the flameless candle.
Figure 14 is a perspective view of a cover that can be removably installed onto the base of Figure 2.

### DETAILED DESCRIPTION OF CERTAIN EMBODIMENTS

Various improved fragrance dispensers, systems, and methods are disclosed herein. The disclosed embodiments are described in connection with a flameless candle, due to particular utility in that context. However, the technology disclosed herein can also be applied to other types of devices and in other contexts. For example, some or all of the subject matter disclosed herein can be used in other types of illumination devices or decor, such as electric lamps, vases, soap dispensers, or otherwise. The discussed components can be added, removed, and/or rearranged. The disclosure herein of any particular feature, aspect, method, property, characteristic, quality, attribute, element, or the like in connection with various embodiments can be used in all other embodiments set forth herein. Any methods described herein may be practiced using any device suitable for performing the recited steps. No feature, element, or step is essential or critical.

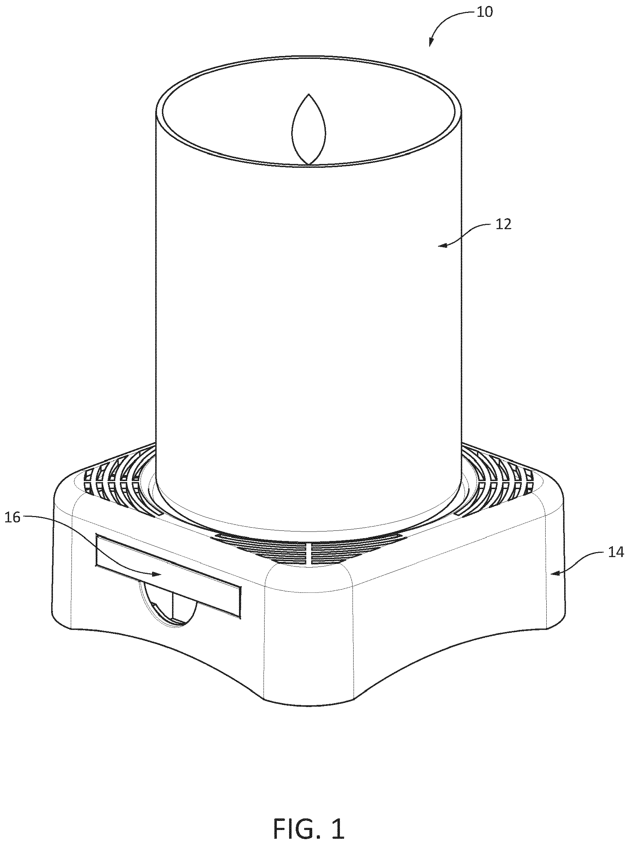

Figure 1 illustrates a perspective view of a fragrance dispenser system 10. The system 10 can include an article to be supported, such as a flameless candle 12. The flameless candle 12 can be, for example, as disclosed in the U.S. Patent 9,341,342 and/or U.S. Patent 11,209,141, the entirety of each of which is hereby incorporated by reference. The system 10 can include a base 14, which is configured to support the article to be supported. For example, as shown, the flameless candle 12 can rest on the base 14. The base 14 can be a discrete and/or self-contained component from the flameless candle 12. In several implementations, the base 14 can operate without the flameless candle 12 and/or the flameless candle 12 can operate without the base 14. Various embodiments of the base 12 do not inhibit illumination provided by the flameless candle 12. As discussed in more detail below, the base 14 can include a tray 16.

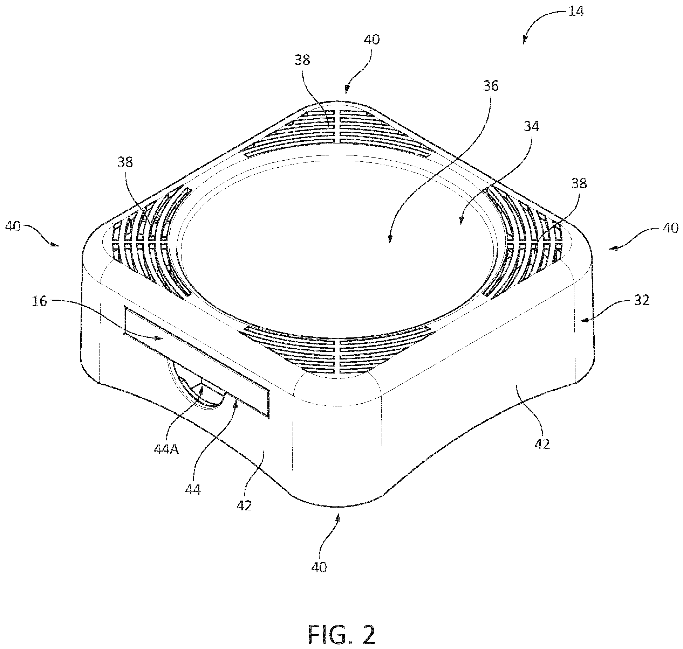

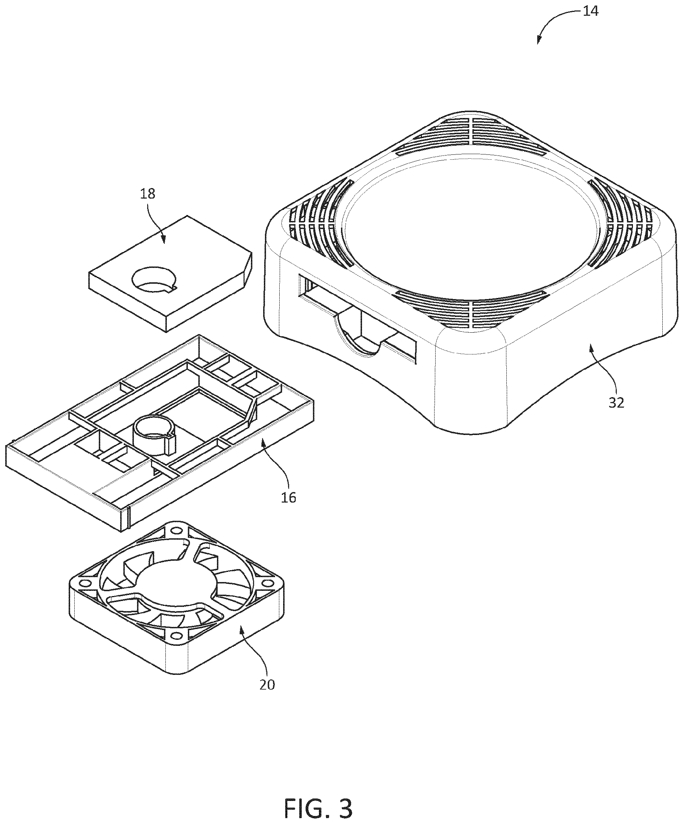

Figures 2, 3, and 4 illustrate aspects of the base 14. The base 14 can include an outer cover 32. The outer cover 32 can be configured to contain and protect various elements of the fragrance dispenser system 10. As further described below, in some embodiments, the outer cover 32, encloses a middle plate and base plate.

The outer cover 32 can have a top surface 34. The top surface 34 can have a recess 36 configured to receive a bottom of the flameless candle 12. The recess 36 can comprise an impression and/or be positioned in the center of the top surface 34. In some embodiments, the recess 36 is a planar region. The recess 36 can be variously shaped, such as circular, square, rectangular, or otherwise. The recess 36 can be sized to accommodate a circular flameless candle, such as flameless candle with a diameter of at least about: 2.2 inch, 3 inch, 3.5 inch, 4 inch, 4.5 inch, or more. The recess 36 can center the flameless candle 12 on the base 14 and/or can reduce the chance of the flameless candle 12 tilting or falling from the base 14.

The outer cover 32 can include one or more outlet vents 38 (also called exhaust vents). The outlet vents 38 can allow fragranced air to exit the base 14, as discussed in more detail below. As shown, in some embodiments, the outlet vents 38 are located in one or more (e.g., each) of the corners 40 of the outer cover 32. The outlet vents 38 can be in the top surface 34. In some embodiments, the outlet vents 38 are not visible from a front and/or side elevation view of base 14.

The outer cover 32 can include sidewalls 42 (also called side panels) that extend downward from the top surface 32. As illustrated, the sidewalls 42 can have corners that extend substantially completely down such that they can make, or nearly make, contact with the surface on which the base 14 is placed. A horizontal edge of each side panel 42 between each corner can be shaped, such as scalloped, to aid in airflow to an intake vent located on a bottom of the base 14.

The base 14 can include an opening 44 for receiving the tray 16. The opening 44 can be located in one of the sidewalls 42. The tray 16 can be configured to slide out to enable a user to insert a fragrance cartridge 18 into the tray 16. In some embodiments, a finger hole 44A is positioned immediately below the fragrance tray 16 to allow the tray 16 to be pulled out.

In several embodiments, the base 14 can include a fan unit 20. The fan unit 20 can include a fan and motor operably connected to the fan. The motor can comprise an electric motor. The fan unit 20 can be housed in the outer cover 32. In several implementations, the fan unit 20 is positioned under the tray 16 and/or is configured to blow air upward onto the cartridge 18 in the tray 16.

In some embodiments, the base 14 includes one or more support structures. For example, the base 14 can include one or more plates that support other components of the base 14, such as the fan and motor. As used herein, "plate" is a broad term that can include a flat or non-flat structure of plastic, metal, or another material that is received in the outer cover 32 and configured to support and/or protect fragrance dispensing components, such as the tray 16, cartridge 18, fan unit 20, batteries 24, PCB 28, or otherwise. The base 14 can include a base plate 50 (alternatively called a base structure) and/or a middle plate 52 (alternatively called a middle structure). The base and middle plates 50, 52 can be coupled together and/or to the outer cover 32. The base plate 50 and/or middle plate 52 can include one or more ribs to increase strength and/or rigidity. Certain variants include only the base plate 50 or the middle plate 52, or neither. For example, in some variants, components of the base 14 can be mounted to the outer cover 32.

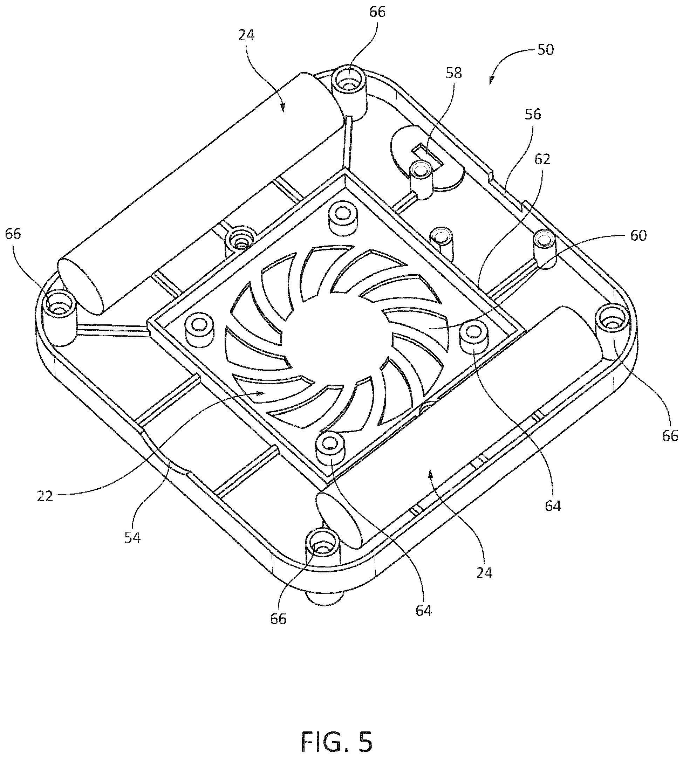

Figure 5 illustrates an example of the base plate 50. The base plate 50 can have a grasping element, such as a finger hole 54, such as in a front side panel. The finger hole 54 can correspond to the finger hole 54 in the outer cover 32. The base plate 50 can have, such as on a back panel, a slot 56 to allow access to a power supply port. The base plate 50 can have, such as on a bottom panel, a power switch slot 58 to accommodate a power switch. A power supply, such as batteries 24 (e.g., rechargeable batteries) can be located on the sides. The base plate 50 can have one or more inlet vents 60. For example, as shown, the base plate 50 can have an air intake vent 60 in the center of the bottom panel. The air intake vent 60 can be in a central portion 22 of the base plate 50.

The base plate 50 can be configured to couple with the fan unit 20. For example, as shown, the base plate 50 can include a chamber 62 with a peripheral wall. The chamber 62 can be in and/or can bound the central portion 22. The chamber 62 can receive a portion (e.g., a bottom end) of the fan unit 20. The chamber 62 can include positioning features, such as bosses 64 that can be received in corresponding recesses in the fan unit 20. In some embodiments, the chamber 62 is centrally located in the base plate 50.

The base plate 50 can be configured to couple to the outer cover 32. For example, as illustrated, the base plate 50 can have connecting tubes 66 that allow a fastener (e.g., a screw) to pass through to secure the base plate 50 to the outer cover 32. The connecting tubes 66 can be located on corners of the base plate 50. In some implementations, the connecting tubes 66 can act as stand offs to elevate the base 14 to help ensure air flow to the air intake vent 60.

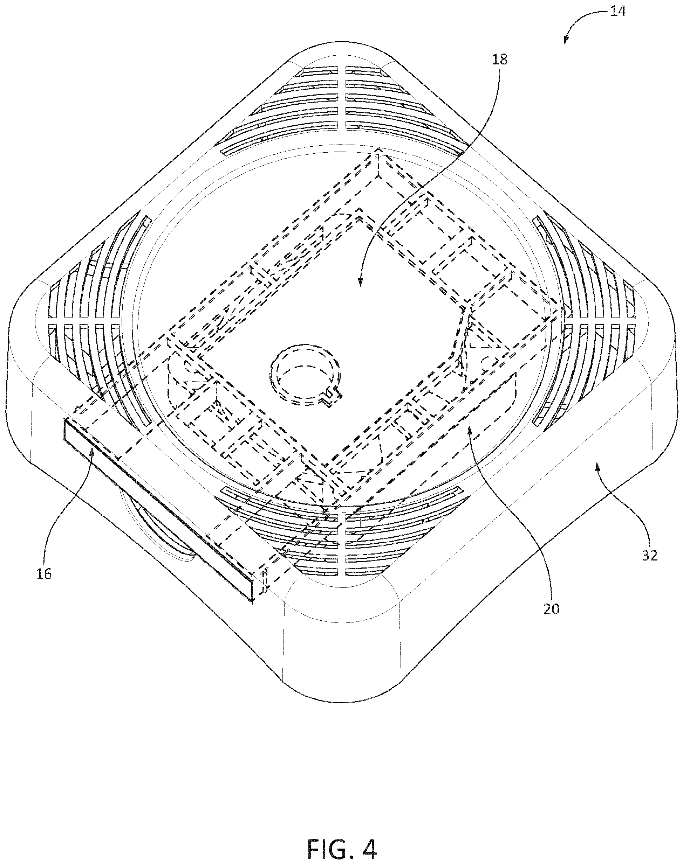

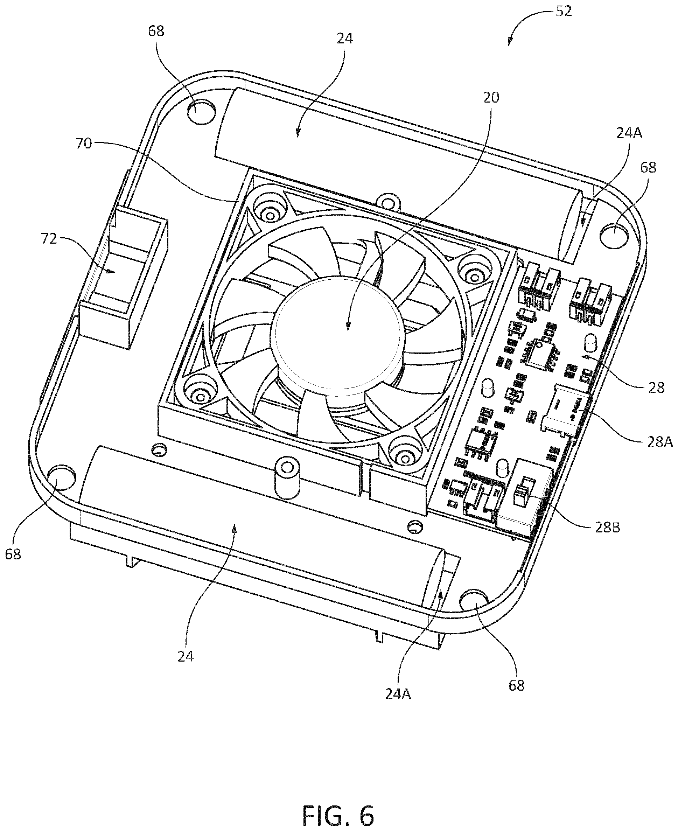

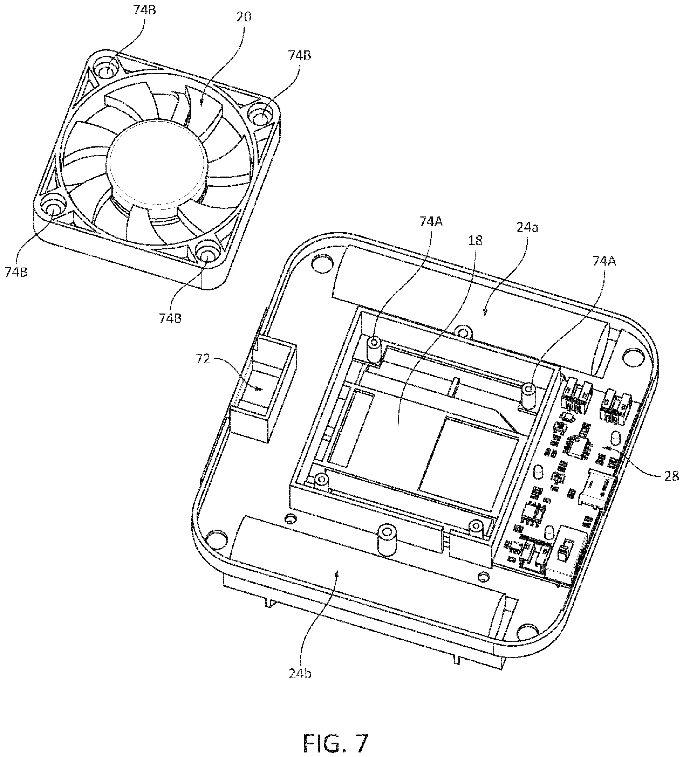

Figures 6 and 7 illustrate an example of the middle plate 52 and associated components. For purposes of presentation, the underside of the middle plate 52 is shown. The underside of the middle plate 52 (Figure 5) can mate with the base plate 50 (Figure 4) to form a support assembly. For example, as shown, the middle plate 52 can have holes 68, such as on corners. The holes 68 can correspond with the connecting tubes 66 of the base plate 50. This can allow the fasteners that connect the base plate 50 and the outer cover 32 to pass through the middle plate 52. In some embodiments, the connecting tubes 66 on each corner of the base plate 50 can fit into the holes 68 in each corner of the middle plate 52.

The fan unit 20 can be coupled to the middle plate 52. For example, as illustrated, the fan unit 20 can be secured within an inner housing 70 with a peripheral wall. In certain implementations, the inner housing 70 includes bosses 74A that mate with corresponding apertures 74B in the fan unit 20. In some implementations, the inner housing 70 and/or the fan unit 20 can be positioned in the center of the middle plate 52. As shown, in some embodiments, the batteries 24 fit into cavities 24A on the sides of the fan unit 20. This can reduce the size (e.g., height) of the support assembly.

As illustrated, the middle plate 52 can house the PCB 28, such as on a back side or elsewhere. The PCB 28 can include a power port 28A and power switch 28B. The power port 28A can extend through and/or be correspondingly located with the slot 56 of the base plate 50, such as to allow an external cord to be plugged in. The power switch 28B can extend through and/or be correspondingly located with the power switch slot 58 of the base plate 50 to enable ready access to the power switch 28B. Conductors, such as wires (not shown), can connect the PCB 28 to the fan unit 20 and/or the batteries 24.

As shown in Figure 7, the middle plate 52 can include a central opening in alignment with the tray 16. As further described below, the fan can blow air (e.g., upward) through the opening, into tray 16, and around and/or through the fragrance cartridge 18. As also shown in Figure 7, the middle plate 52 can include a wall 72. The wall 72 can at least partly form a cavity that is in communication with the finger hole 44A. The user can insert a finger into the cavity via the finger hole 44A to remove the tray 16.

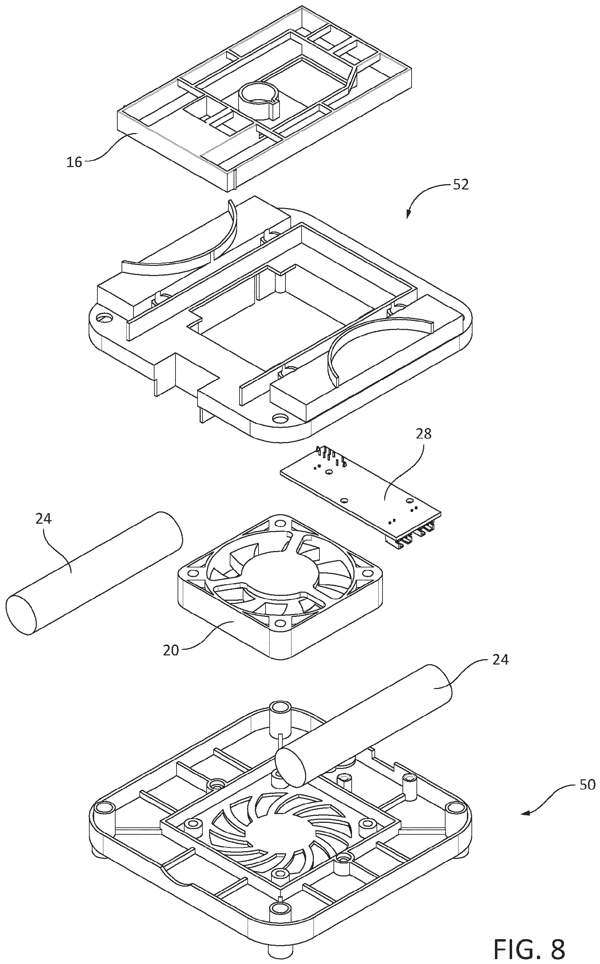

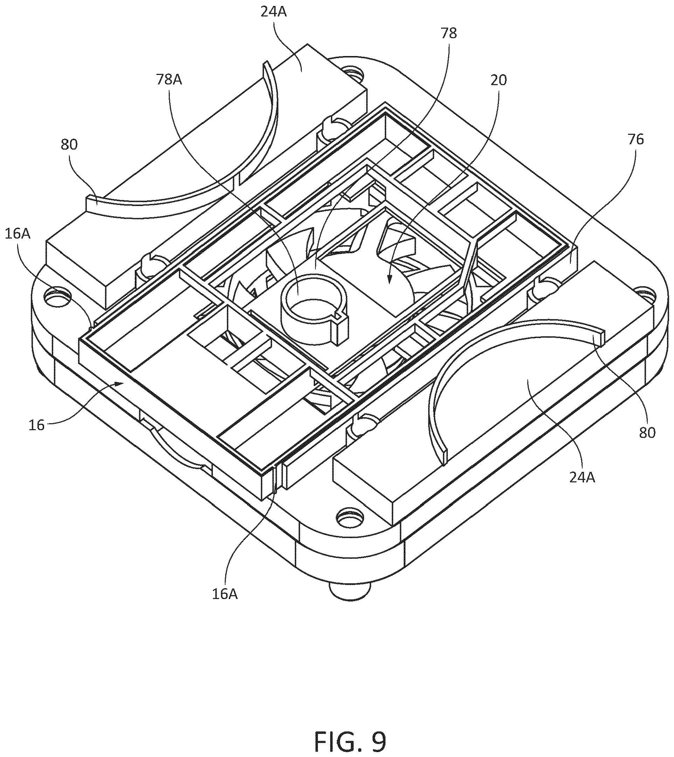

Figures 8 and 9 illustrate the support assembly (including the base plate 50 and middle plate 52) and the tray 16. The middle plate 52 can be configured to receive the tray 16. For example, the middle plate 52 can include a tray retention wall 76. The wall 76 can be positioned in the center of the middle plate 52 to securely locate the fragrance tray 16 over the fan unit 20 and air intake (not shown). The wall 76 can be supported by the battery cavities 24A, which can be located on long sides of the wall 76, as shown. The fragrance tray 16 can fit within, and be retained in, the space outlined by the fragrance tray retention wall 76. The tray 16 can have stops 16A that limit insertion of the tray 16 into the base 14. For example, the stops 16A can abut the wall 76.

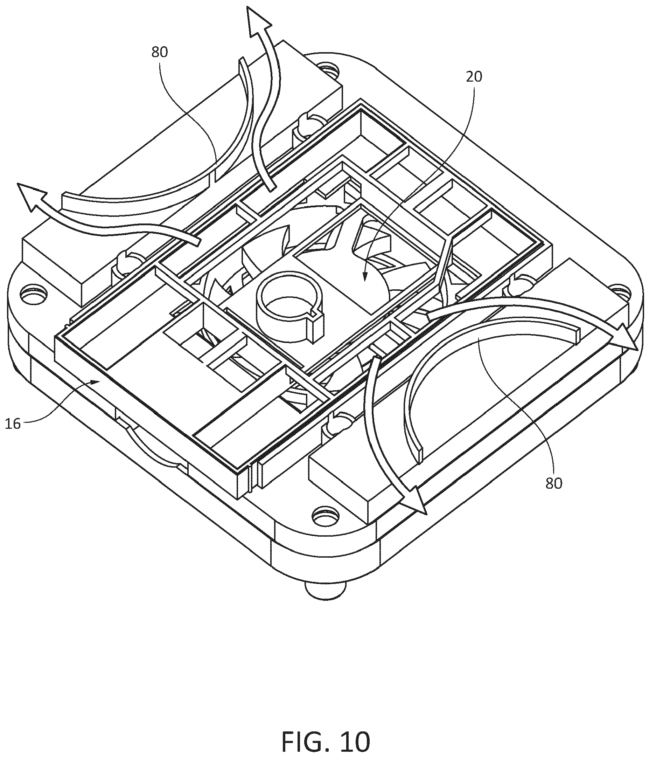

The fragrance tray 16 can have an opening 78 to accept the fragrance cartridge 18 (see Figures 10-13). In some embodiments, the opening 78 can have features configured to properly orient the fragrance cartridge 18. For example, certain implementations have a center peg 78A that is offset towards the front side of the fragrance tray 16, a positioning notch 78B that extends to one side of the center peg 78A, and/or a clipped corner 78C, such as a chamfer. The features can ensure that the fragrance cartridge 18 can only be positioned in the correct orientation in the tray 16.

As schematically shown in Figure 10, the fan unit 20 can blow air through the tray 16. As also shown, the battery cavities 24A can each include a deflector vane 80. The deflector vane 80 can be curved or otherwise shaped. The vane 80 can be configured to direct air flow leaving the tray 16 toward the corners of the base 14 and/or the outlet vents 38.

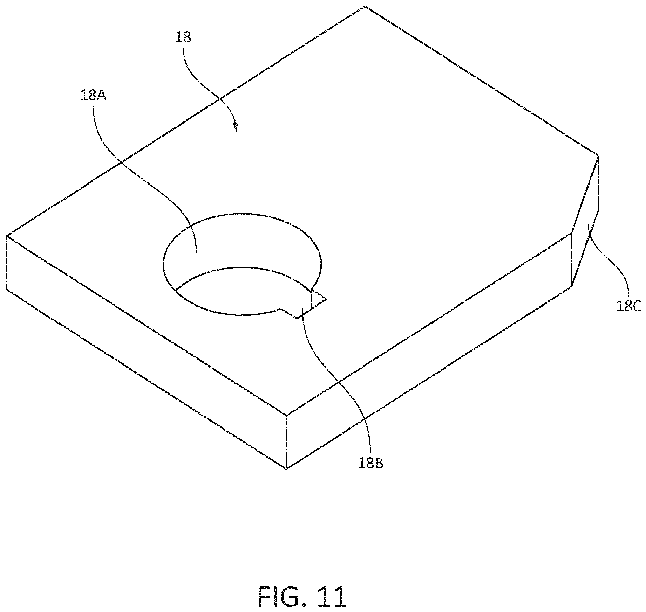

Figure 11 illustrates an example of the fragrance cartridge 18. As previously discussed, the fragrance cartridge tray 16 can have multiple features to ensure that the fragrance cartridge 18 can only be positioned in the tray 16 in the correct orientation. The cartridge 18 can have corresponding and/or mating features. For example, the cartridge 18 can have a center peg opening 18A that is offset towards the front side of the cartridge 18, a positioning notch opening 18B that extends to one side of the center peg opening 18A, and/or a clipped corner notch 18C on a rear corner of the cartridge 18.

In several embodiments, the fragrance cartridge 18 comprises a solid material. A solid fragrance cartridge can be easier and/or less messy to handle and install compared to a fragrant liquid or a cartridge including fragrant liquid. In certain embodiments, the fragrance cartridge 18 can be made of diatomaceous earth, scented polymer, a porous membrane, wicking fiber, or otherwise. In some implementations, the solid material is impregnated with a scented liquid. The fragrance cartridge 18 can be or include a non-solid material, such as gas or liquid. In some implementations, the fragrance cartridge 18 can be made of a fabric material, such as felt, cotton, wool, or otherwise. In certain variants, the fragrance cartridge 18 can be made of a plastic tray with a permeable film to seal a liquid fragrance. Before use in the tray 16, the fragrance cartridge 18 can be stored separately in an outer pouch, such as a foil pouch. The pouch can protect the fragrance cartridge 18 and reduce loss of fragrance.

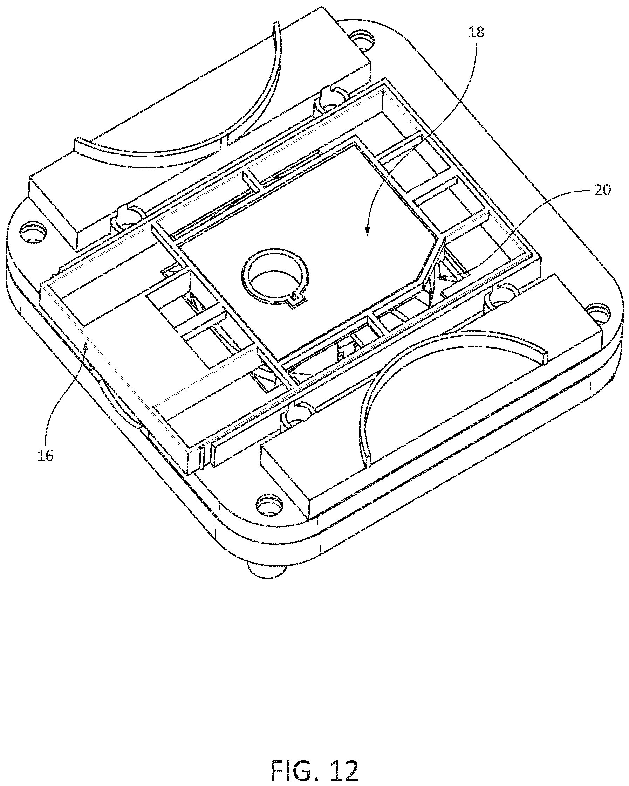

As shown in Figure 12, the cartridge 18 can be securely received in the tray 16. The tray 16 can include openings on one or more sides of the cartridge 18. The fan unit 20 can blow air through the tray 16 and around the cartridge 18, thereby producing fragranced air. The fragranced air can be routed, such as with the vanes 80, to the outlet vents 38.

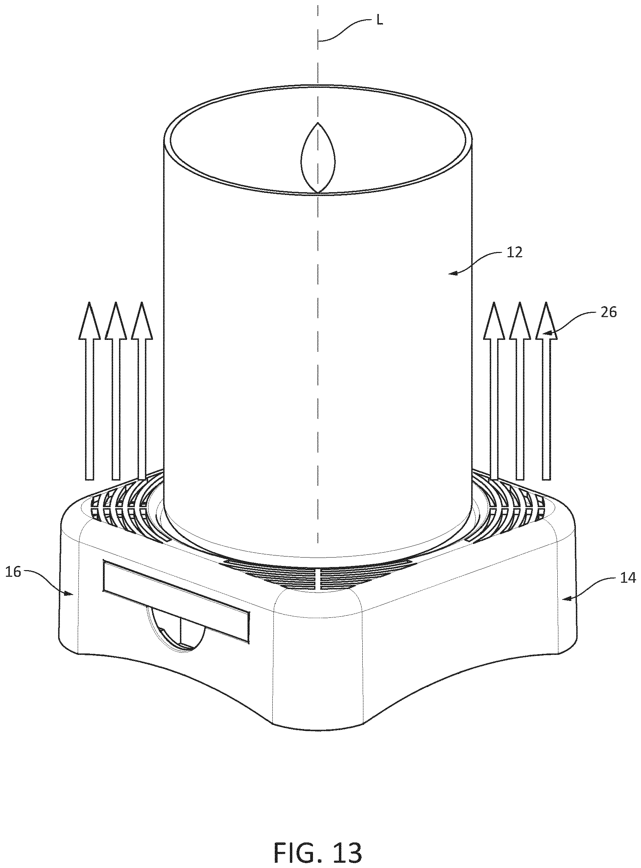

During operation, air can be drawn into the base 14, by the fan unit 20, through the air inlet vent 60. The air can pass through the support assembly, such as through a center of the support assembly. The fan unit 20 can move the air (e.g., upward) into the tray 16. For example, the air can be blown through the opening in the middle plate 52. The air can pass through apertures in the tray 16. At least some of the aid can pass through the tray 16. The air can pass around and/or through the fragrance cartridge 18 to the tray 16. This fragrances the air, thereby producing fragranced air. The fragranced air can be directed toward the outlet vents 38. For example, the fragranced air can be directed by the vanes 80. The fragranced air can be discharged from the base 14 through the outlet vents 38 and into the ambient environment. As shown in Figure 13, the outlet vents can be distributed (e.g., approximately equally spaced) around the base 14 and/or the periphery of the flameless candle 12, which can facilitate generally even fragrance distribution in the vicinity of the fragrance dispenser system.

As also shown in Figure 13, the fragranced air can be discharged from the outlet vents 38 in an upward direction. This can, for example, facilitate distribution of the fragranced air in the room. As illustrated, the air can be discharged in a direction that is generally parallel to a longitudinal axis L of the flameless candle 12. The air can be discharged in a direction that is toward a top of the flameless candle 12. The air can be discharged in a direction that is generally perpendicular to a planar region of the base 14, on which the flameless candle 12 rests. In certain embodiments, the air is not discharged from the sidewall 42 and/or in a direction laterally outward.

In some embodiments, the base 14 includes a heater (not shown). The heater can comprise, for example, an electric resistance heater. The heater can receive electrical power from the power supply 24. Operation of the heater, such as the period of heating and/or temperature of heating, can be controlled by the PCB 28. In some implementations, the heater is configured to heat the fragrance cartridge 18. In some implementations, the heater is configured to heat the air before and/or after the air encounters the fragrance cartridge 18. For example, the heater can heat the air after the air enters the inlet vent 60, after or while the air passes through the fan unit 20, and/or before the air enters the tray 16.

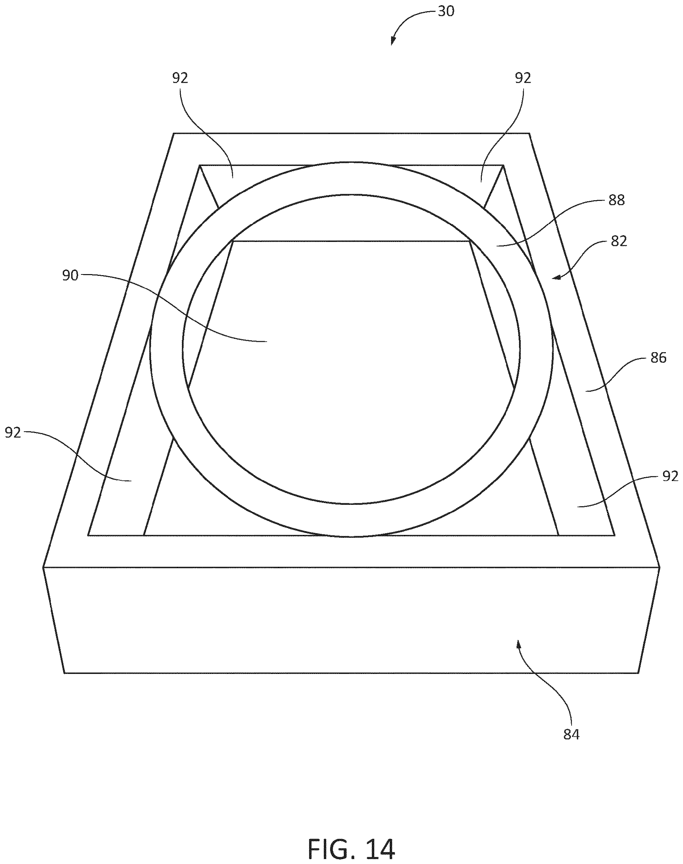

In some implementations, the fragrance dispenser system 10 includes a cover 30, such as is illustrated in Figure 14. The cover 30 can be configured to be removably installed onto the base 14. For example, the cover 30 can be installed over the outer cover 32 of the base 14. The cover 30 can partially or completely mask the base 14 from view and can provide a different visual appearance than the base 14. The cover 30 can enable a user to readily change the appearance of the base 14 (and the fragrance dispenser system 10) simply by adding or changing the cover 30. For example, in the winter a cover 30 with a winter theme may be added onto the base 14, in the spring a cover 30 with a spring theme may be added onto the base 14, etc. The cover 30 can be made of plastic, metal, wood, ceramic, or any other suitable material.

The cover 30 can have an upper surface 82 and a cover wall 84. As illustrated, the cover wall 82 can extend downward from the upper surface 82. When the cover 30 is installed over the base 14, the cover wall 84 overlaps the sidewall 42 of base 14. The cover wall 84 can thus block view of the sidewall 42. The cover 30 can be hollow. The cover 30 can be configured to removably secure to the base 14, such as with clips, latches, detents, or otherwise. For example, the cover wall 84 can include deflectable fingers that resiliently latch to a bottom edge of the outer cover 32 of the base14.

The upper surface 82 can be configured to cooperate with features of the base 14. The upper surface 82 can have a peripheral portion 86, which cooperates with an upper periphery of the base 14. For example, as illustrated, the peripheral portion 86 can be generally square, though other shapes are contemplated as well, such as circular or otherwise. The upper surface 82 can have an inner portion 88, which cooperates with the recess 36 of the base 14. For example, as illustrated, the inner portion 88 can be generally circular, though other shapes are contemplated as well, such as rectangular or otherwise.

The upper surface can include one or more apertures. In some embodiments, the inner portion 88 includes an aperture 90, such that the bottom of the flameless candle 12 can be passed through the aperture 90 and received in the recess 36 on the base 14. In certain embodiments, the upper surface 82 includes one or more apertures 92 that cooperate with the one or more outlet vents 38 of the base 14, such that the air blown out of the outlet vents 38 passes through the cover 30. As shown, the apertures 92 can be formed between the peripheral and inner portions of the upper surface 82.

Terms of orientation used herein, such as "top," "bottom," "horizontal," "vertical," "longitudinal," "lateral," and "end" are used in the context of the illustrated embodiment. However, the present disclosure should not be limited to the illustrated orientation. Indeed, other orientations are possible and are within the scope of this disclosure. Terms relating to circular shapes as used herein, such as diameter or radius, should be understood not to require perfect circular structures, but rather should be applied to any suitable structure with a cross-sectional region that can be measured from side-to-side. Terms relating to shapes generally, such as "circular" or "cylindrical" or "semi-circular" or "semi-cylindrical" or any related or similar terms, are not required to conform strictly to the mathematical definitions of circles or cylinders or other structures, but can encompass structures that are reasonably close approximations.

Conditional language used herein, such as, "can," "could," "might," "may," "e.g.," and the like, unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment.

Conjunctive language, such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require at least one of X, at least one of Y, and at least one of Z to each be present.

The terms "comprising," "including," "having," and the like are synonymous and are used inclusively, in an open-ended fashion, and do not exclude additional elements, features, acts, operations and so forth. Also, the term "or" is used in its inclusive sense (and not in its exclusive sense) so that when used, for example, to connect a list of elements, the term "or" means one, some, or all of the elements in the list. The term "and/or" means that "and" applies to some embodiments and "or" applies to some embodiments. Thus, A, B, and/or C is equivalent to A, B, and C written in one sentence and A, B, or C written in another sentence. The term "and/or" is used to avoid unnecessary redundancy.

The terms "approximately," "about," and "substantially" as used herein represent an amount close to the stated amount that still performs a desired function or achieves a desired result. For example, in some embodiments, as the context may dictate, the terms "approximately", "about", and "substantially" may refer to an amount that is within less than or equal to 10% of the stated amount. The term "generally" as used herein represents a value, amount, or characteristic that predominantly includes or tends toward a particular value, amount, or characteristic. As an example, in certain embodiments, as the context may dictate, the term "generally parallel" can refer to something that departs from exactly parallel by less than or equal to 20 degrees and the term "generally perpendicular" can refer to something that departs from exactly perpendicular by less than or equal to 20 degrees.

Various embodiments and examples of fragrance dispensers, systems, and methods have been disclosed. Although the disclosure has been in the context of those embodiments and examples, this disclosure extends beyond the specifically disclosed embodiments to other alternative embodiments and/or other uses of the embodiments, as well as to certain modifications and equivalents thereof. Moreover, this disclosure expressly contemplates that various features and aspects of the disclosed embodiments can be combined with, or substituted for, one another. Accordingly, the scope of this disclosure should not be limited by the particular disclosed embodiments described herein.

## Claims

1. A fragrance dispenser system comprising:
a flameless candle having a longitudinal axis and a bottom; and
a base configured to receive and support the flameless candle, the base comprising:
an upper surface comprising a recess and a plurality of outlet vents, the recess shaped to correspond to a shape of the bottom of the flameless candle;
a lower surface comprising an inlet vent;
a sidewall connecting the upper surface and the lower surface, the sidewall comprising a tray;
a fragrance cartridge configured to be removably received in the tray;
a fan positioned beneath the tray;
an electric motor operably connected with the fan; and
a printed circuit board configured to control the electric motor;
wherein the base is configured to draw air into the base through the inlet vent, to pass the air through the tray, and to dispense the air out of the base through the plurality of outlet vents in a direction generally parallel to the longitudinal axis of the flameless candle.

2. The fragrance dispenser system of claim 1, wherein the flameless candle comprises an LED candle.

3. The fragrance dispenser system of claim 1, further comprising a power source.

4. The fragrance dispenser system of claim 3, wherein the power source comprises a battery.

5. The fragrance dispenser system of claim 1, wherein the sidewall comprises an opening and the tray is configured to slide through the opening between opened and closed positions.

6. The fragrance dispenser system of claim 1, further comprising a cover configured to removably engage the base.

7. The fragrance dispenser system of claim 6, wherein the cover comprises one or more clips to secure the cover to the base.

8. The fragrance dispenser system of claim 1, wherein the printed circuit board is programmed to turn on and off the electric motor.

9. The fragrance dispenser system of claim 1, wherein the printed circuit board is programmed to control an interval time of operation of the electric motor.

10. The fragrance dispenser system of claim 1, further comprising a heater configured to heat the fragrance cartridge, and wherein the printed circuit board is programmed to control the heater.

11. The fragrance dispenser system of claim 10, wherein the fragrance cartridge is a solid.

12. A fragrance dispenser system configured to support a flameless candle, the fragrance dispenser system comprising:
a base comprising:
an upper surface comprising a planar region and a plurality of outlet vents, the planar region configured to support the flameless candle, the plurality of outlet vents configured to blow air toward a top of the flameless candle;
a lower surface comprising an inlet vent;
a sidewall connecting the upper surface and the lower surface, the sidewall comprising an opening;
a tray configured to slide in and out of the opening;
a fan;
an electric motor operably connected with the fan; and
a printed circuit board configured to control the electric motor; and
a solid fragrance cartridge configured to be removably received in the tray.

13. The fragrance dispenser system of claim 12, further comprising a heater configured to heat the solid fragrance cartridge.

14. The fragrance dispenser system of claim 12, wherein the fan is positioned beneath the tray.

15. The fragrance dispenser system of claim 12, wherein the base is configured to support a flameless candle having a diameter of at least 3 inches.

16. The fragrance dispenser system of claim 12, wherein the inlet vent is configured to receive air from below the fragrance cartridge and the fan is configured to blow air upward onto the solid fragrance cartridge positioned in the tray.

17. A fragrance dispenser system configured to receive a flameless candle, the fragrance dispenser system comprising:
a base comprising:
an upper surface comprising a receiving region and an outlet vent, the receiving region configured to receive a bottom of the flameless candle;
a lower surface comprising an inlet vent;
a sidewall connecting the upper surface and the lower surface, the sidewall comprising an opening;
a tray configured to be received in the opening;
a fan configured to blow air out of the outlet vent;
an electric motor operably connected with the fan; and
a controller configured to control operation of the motor;
a fragrance cartridge configured to be received in the tray; and
a cover configured to be removably installed over the base, the cover comprising:
an upper surface comprising a first aperture and a second aperture; and
a cover wall that extends downward from the upper surface;
the cover configured such that, when the cover is installed over the base:
the cover wall of the cover overlaps the sidewall of base, such that the cover wall blocks view of the sidewall;
the first aperture of the cover corresponds to the receiving region of the base, such that the bottom of the flameless candle can be passed through first aperture and received on the receiving region; and
the second aperture of the cover corresponds to the outlet vent of the base, such that the air blown out of the outlet vent passes through the second aperture.

18. The fragrance dispenser system of claim 17, wherein the fragrance cartridge comprises a solid fragrance cartridge.

19. The fragrance dispenser system of claim 17, wherein the fragrance cartridge comprises diatomaceous earth.

20. The fragrance dispenser system of claim 17, wherein the fragrance cartridge comprises a circular hole with a notch to one side.
